# EUROPEAN PATENT SPECIFICATION

(11) **EP 3 801 524 B1**
(45) Date of publication and mention of the grant of the patent: **05.11.2025**
(21) Application number: 19810149.5
(22) Date of filing: 30.05.2019
(51) Int. Cl.: A61K 31/496, A61K 31/506, A61P 35/00

(54) **COMPOSITION AND METHOD OF TREATING CANCER ASSOCIATED WITH EGFR MUTATION**
ZUSAMMENSETZUNG UND VERFAHREN ZUR BEHANDLUNG VON KREBS IN ZUSAMMENHANG MIT EINER EGFR-MUTATION
COMPOSITION ET PROCÉDÉ DE TRAITEMENT DU CANCER ASSOCIÉ À UNE MUTATION EGFR

(30) Priority: 01.06.2018 US 201862679619 P
(43) Date of publication of application: 14.04.2021
(73) Proprietor: Beyondspring Pharmaceuticals, Inc., 07932 New Jersey (US)
(72) Inventor: HUANG, Lan, New York, New York 10005 (US); LLOYD, George, Kenneth, New York, New York 10005 (US)
(74) Representative: Callaghan, Dayle Anne
(86) International application number: PCT/US2019/034726
(87) International publication number: WO 2019/232257

(56) References cited:
- WO-A1-2011/034954
- WO-A1-2014/130657
- WO-A1-2016/130839
- WO-A1-2016/144635
- WO-A1-2016/144635
- WO-A1-2018/169887
- WO-A2-2004/054498
- MOHANLAL R W ET AL: "Plinabulin as a novel small molecule clinical stage immuno-oncology agent for NSCLC.", JOURNAL OF CLINICAL ONCOLOGY 20170301 AMERICAN SOCIETY OF CLINICAL ONCOLOGY NLD, vol. 35, no. 7, Supplement 1, 1 March 2017 (2017-03-01), XP002805352, ISSN: 1527-7755
- LIU HAIJING ET AL: "Spectrum of EGFR aberrations and potential clinical implications: insights from integrative pan-cancer analysis", CANCER COMMUNICATIONS, vol. 40, no. 1, 31 December 2019 (2019-12-31), pages 43 - 59, XP93140837, ISSN: 2523-3548, Retrieved from the Internet <URL:https://www.ncbi.nlm.nih.gov/pmc/articles/PMC7163653/pdf/CAC2-40-43.pdf> DOI: 10.1002/cac2.12005
- "NCT02846792 Nivolumab and Plinabulin in Treating Patients With Stage IIIB-IV, Recurrent, or Metastatic Non-small Cell Lung Cancer", CLINICALTRIALS.GOV, vol. NCT02846792, July 2016 (2016-07-01), pages 1 - 10, XP009524744, Retrieved from the Internet <URL:https://clinicaltrials.gov/ct2/show/NCT02846792> [retrieved on 20170122]
- MUGURUMA, K.; KAWAMATA, R.; AKIYAMA, D.; ARIMA, R.; SHIRASAKA, T. ; KIKKAWA, A.; TAGUCHI A.; TAKAYAMA, K.; FUKUHARA, T.; ITO, Y.; : "Application of Fc-selective Z33-peptide to the preparation of non-covalent-type antibody-antimicrotubule plinabulin conjugate", JOURNAL OF PEPTIDE SCIENCE, vol. 22, no. 52, September 2016 (2016-09-01), pages S19, XP009524709, ISSN: 1099-1387
- YANG, MING-JE ET AL.: "The KRAS Mutation is Highly Correlated With EGFR Alterations in Patients With Non-small Cell Lung Cancer", FOOYIN JOURNAL OF HEALTH SCIENCES, vol. 1, no. 2, 2009, pages 65 - 71, XP026944905

## Description

### BACKGROUND OF THE INVENTION

### Field

The present invention relates to the field of chemistry and medicine. More particularly, the present invention relates to method of treating cancer associated with epidermal growth factor receptor. The invention is defined in the appended claims.

### Description of the Related Art

Enzymes capable of catalyzing the phosphorylation reactions of proteins on tyrosine residues are termed tyrosine kinases. A number of transmembrane receptors contain domains with tyrosine kinase activity and are classified as receptor tyrosine kinases (RTKs). There are several members of this family of RTKs, class I of which includes the erbB family, e.g. epidermal growth factor receptor (EGFR), erbB2, erbB3 and erbB4. The EGFR tyrosine kinase domain is activated by binding of a variety of ligands to the external domain.

The erbB family of receptor tyrosine kinases are known to be frequently involved in driving the proliferation and survival of tumor cells. One mechanism by which this can occur is over expression of the receptor at the protein level, for example as a result of gene amplification. This has been observed in many common human cancers such as, non-small cell lung cancers (NSCLCs) including adenocarcinomas as well as other cancers of the lung. EGFR is markedly overexpressed across a large variety of epithelial cancers and some immunohistochemical studies have demonstrated EGFR expression is associated with poor prognosis. There is a need to develop drugs that can treat EGFR related cancers. Mohanlal R.W. et al. (Journal of Clinical Oncology (2017), 35(7 Suppl.), 139) reports a phase 2 study using plinabulin alone or in combination with docetaxel in NSCLC patients and a synergy with PD-1 or CTLA-4 inhibitors.

### SUMMARY

Some embodiments relate to plinabulin, or a pharmaceutically acceptable salt thereof, for use in combination with osimertinib for use in a method of treating a cancer or tumor characterized by expression of a mutant form of an epidermal growth factor receptor (EGFR) protein, wherein the mutant form of the EGFR protein comprises an amino acid substitution T790M.

Some embodiments relate to plinabulin, or a pharmaceutically acceptable salt thereof, for use in combination with osimertinib for use in a method of inhibiting proliferation of a cell having an EGFR mutation or a cell expressing a mutant form of an EGFR protein, wherein the mutant form of the EGFR protein comprises an amino acid substitution T790M.

Some embodiments relate to plinabulin, or a pharmaceutically acceptable salt thereof, for use in combination with osimertinib for use in a method of inhibiting progression of a cancer characterized by expression of a mutant form of an EGFR protein in a subject, wherein the mutant form of the EGFR protein comprises an amino acid substitution T790M.

Some embodiments relate to a pharmaceutical composition comprising plinabulin and osimertinib.

### BRIEF DESCRIPTION OF THE DRAWINGS

Figure 1 shows the change of tumor sizes in the six tested groups.
Figure 2a shows the change of tumor size on day 6; Figure 2b shows the change of tumor size on day 9; Figure 2c shows the change of tumor size on day 13; and Figure 2d shows the plinabulin dose response curve on day 24 in EGFR mutant mice.

### DETAILED DESCRIPTION OF THE PREFERRED EMBODIMENT

Plinabulin, (3*Z*,6*Z*)-3-Benzylidene-6-{[5-(2-methyl-2-propanyl)-1*H-*imidazol-4-yl]methylene}-2,5-piperazinedione, is a synthetic analog of the natural compound phenylahistin. Plinabulin can be readily prepared according to methods and procedures detailed in U.S. Patents 7,064,201 and 7,919,497. The invention is defined in the appended claims. Some embodiments relate to the use of Plinabulin to treat cancer associated with an oncogenic EGFR mutation. Some embodiments relate to the use of Plinabulin to treat a cancer characterized by expressing a mutant form of EGFR protein in a subject. Some embodiments relate to the use of Plinabulin to inhibit proliferation of a cell having an EGFR mutation. Some embodiments relate to the use of Plinabulin to induce apoptosis in a cell having an EGFR mutation. Some embodiments relate to the use of Plinabulin to inhibit progression of a cancer that is characterized by expressing a mutant form of EGFR protein in a subject.

Unless defined otherwise, all technical and scientific terms used herein have the same meaning as is commonly understood by one of ordinary skill in the art to which this disclosure belongs. All patents, applications, published applications, and other publications are incorporated by reference in their entirety. In the event that there is a plurality of definitions for a term herein, those in this section prevail unless stated otherwise.

"Subject" as used herein, means a human or a non-human mammal, e.g., a dog, a cat, a mouse, a rat, a cow, a sheep, a pig, a goat, a non-human primate or a bird, e.g., a chicken, as well as any other vertebrate or invertebrate.

The term "mammal" is used in its usual biological sense. Thus, it specifically includes, but is not limited to, primates, including simians (chimpanzees, apes, monkeys) and humans, cattle, horses, sheep, goats, swine, rabbits, dogs, cats, rodents, rats, mice guinea pigs, or the like.

An "effective amount" or a "therapeutically effective amount" as used herein refers to an amount of a therapeutic agent that is effective to relieve, to some extent, or to reduce the likelihood of onset of, one or more of the symptoms of a disease or condition, and includes curing a disease or condition.

"Treat," "treatment," or "treating," as used herein refers to administering a compound or pharmaceutical composition to a subject for prophylactic and/or therapeutic purposes. The term "prophylactic treatment" refers to treating a subject who does not yet exhibit symptoms of a disease or condition, but who is susceptible to, or otherwise at risk of, a particular disease or condition, whereby the treatment reduces the likelihood that the patient will develop the disease or condition. The term "therapeutic treatment" refers to administering treatment to a subject already suffering from a disease or condition.

The term "pharmaceutically acceptable salt" refers to salts that retain the biological effectiveness and properties of a compound and, which are not biologically or otherwise undesirable for use in a pharmaceutical. In many cases, the compounds disclosed herein are capable of forming acid and/or base salts by virtue of the presence of amino and/or carboxyl groups or groups similar thereto. Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, toluene sulfonic acid, citric acid, benzoic acid, cinnamic acid, mandelic acid, methanesulfonic acid, ethanesulfonic acid, p-toluenesulfonic acid, salicylic acid, and the like. Pharmaceutically acceptable salts can also be formed using inorganic and organic bases. Inorganic bases from which salts can be derived include, for example, bases that contain sodium, potassium, lithium, ammonium, calcium, magnesium, iron, zinc, copper, manganese, aluminum, and the like; particularly preferred are the ammonium, potassium, sodium, calcium and magnesium salts. In some embodiments, treatment of the compounds disclosed herein with an inorganic base results in loss of a labile hydrogen from the compound to afford the salt form including an inorganic cation such as Li⁺, Na⁺, K⁺, Mg²⁺ and Ca²⁺ and the like. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. Many such salts are known in the art, as described in WO 87/05297, Johnston et al., published September 11, 1987 (incorporated by reference herein in its entirety).

The term "pharmaceutically acceptable carrier" or "pharmaceutically acceptable excipient" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. In addition, various adjuvants such as are commonly used in the art may be included. Considerations for the inclusion of various components in pharmaceutical compositions are described, e.g., in Gilman et al. (Eds.) (1990); Goodman and Gilman's: The Pharmacological Basis of Therapeutics, 8th Ed., Pergamon Press.

### Pharmaceutical Composition and Administration

Some embodiments relate to a pharmaceutical composition for treating a cancer or tumor characterized by expression of a mutant form of an epidermal growth factor receptor (EGFR) protein, wherein the composition comprises plinabulin and osimertinib.

Some embodiments relate to a pharmaceutical composition comprising plinabulin and osimertinib.

In some embodiments, the composition comprising plinabulin can further include one or more pharmaceutically acceptable diluents. In some embodiments, the pharmaceutically acceptable diluent can include Kolliphor HS 15^{®} (Polyethylene glycol (15)-hydroxystearate). In some embodiments, the pharmaceutically acceptable diluent can include propylene glycol. In some embodiments, the pharmaceutically acceptable diluents can include kolliphor and propylene glycol. In some embodiments, the pharmaceutically acceptable diluents can include kolliphor and propylene glycol, wherein the kolliphor is about 40% by weight and propylene glycol is about 60% by weight based on the total weight of the diluents. In some embodiments, the composition can further include one or more other pharmaceutically acceptable excipients.

Standard pharmaceutical formulation techniques can be used to make the pharmaceutical compositions described herein, such as those disclosed in Remington's The Science and Practice of Pharmacy, 21st Ed., Lippincott Williams & Wilkins (2005). Accordingly, some embodiments include pharmaceutical compositions comprising: (a) a safe and therapeutically effective amount of Plinabulin or pharmaceutically acceptable salts thereof; and osimertinib; and (b) a pharmaceutically acceptable carrier, diluent, excipient or combination thereof.

Other embodiments include co-administering Plinabulin and osimertinib in separate compositions or the same composition. Thus, some embodiments include a first pharmaceutical composition comprising: (a) a safe and therapeutically effective amount of Plinabulin or pharmaceutically acceptable salts thereof and (b) a pharmaceutically acceptable carrier, diluent, excipient or combination thereof; and a second pharmaceutical composition comprising: (a) a safe and therapeutically effective amount of an additional therapeutic agent and (b) a pharmaceutically acceptable carrier, diluent, excipient or combination thereof. Some embodiments include a pharmaceutical composition comprising: (a) a safe and therapeutically effective amount of Plinabulin or pharmaceutically acceptable salts thereof; (b) a safe and therapeutically effective amount of an additional therapeutic agent; and (c) a pharmaceutically acceptable carrier, diluent, excipient or combination thereof.

Administration of the pharmaceutical compositions as described below or elsewhere herein can be via any of the accepted modes of administration for agents that serve similar utilities including, but not limited to, orally, sublingually, buccally, subcutaneously, intravenously, intranasally, topically, transdermally, intradermally, intraperitoneally, intramuscularly, intrapulmonarilly, vaginally, rectally, or intraocularly. Oral and parenteral administrations are customary in treating the indications that are the subject of the preferred embodiments.

Some examples of substances, which can serve as pharmaceutically-acceptable carriers or components thereof, are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and oil of theobroma; polyols such as propylene glycol, glycerin, sorbitol, mannitol, and polyethylene glycol; alginic acid; emulsifiers, such as the TWEENS; wetting agents, such sodium lauryl sulfate; coloring agents; flavoring agents; tableting agents, stabilizers; antioxidants; preservatives; pyrogen-free water; isotonic saline; and phosphate buffer solutions.

The compositions described herein are preferably provided in unit dosage form. As used herein, a "unit dosage form" is a composition containing an amount of a compound or composition that is suitable for administration to an animal, preferably a mammalian subject, in a single dose, according to good medical practice. The preparation of a single or unit dosage form however, does not imply that the dosage form is administered once per day or once per course of therapy. Such dosage forms are contemplated to be administered once, twice, thrice or more per day and may be administered as infusion over a period of time (e.g., from about 30 minutes to about 2-6 hours), or administered as a continuous infusion, and may be given more than once during a course of therapy, although a single administration is not specifically excluded. The skilled artisan will recognize that the formulation does not specifically contemplate the entire course of therapy and such decisions are left for those skilled in the art of treatment rather than formulation.

The compositions useful as described above may be in any of a variety of suitable forms for a variety of routes for administration, for example, for oral, sublingual, buccal, nasal, rectal, topical (including transdermal and intradermal), ocular, intracerebral, intracranial, intrathecal, intra-arterial, intravenous, intramuscular, or other parental routes of administration. The skilled artisan will appreciate that oral and nasal compositions include compositions that are administered by inhalation and made using available methodologies. Depending upon the particular route of administration desired, a variety of pharmaceutically-acceptable carriers well-known in the art may be used. Pharmaceutically-acceptable carriers include, for example, solid or liquid fillers, diluents, hydrotropies, surface-active agents, and encapsulating substances. Optional pharmaceutically-active materials may be included, which do not substantially interfere with the activity of the compound or composition. The amount of carrier employed in conjunction with the compound or composition is sufficient to provide a practical quantity of material for administration per unit dose of the compound. Techniques and compositions for making dosage forms useful in the methods described herein are described in the following references:
Modern Pharmaceutics, 4th Ed., Chapters 9 and 10 (Banker & Rhodes, editors, 2002); Lieberman *et al.,* Pharmaceutical Dosage Forms: Tablets (1989); and Ansel, Introduction to Pharmaceutical Dosage Forms 8th Edition (2004).

Various oral dosage forms can be used, including such solid forms as tablets, capsules (e.g., liquid gel capsule and solid gel capsule), granules and bulk powders. Tablets can be compressed, tablet triturates, enteric-coated, sugar-coated, film-coated, or multiple-compressed, containing suitable binders, lubricants, diluents, disintegrating agents, coloring agents, flavoring agents, flow-inducing agents, and melting agents. Liquid oral dosage forms include aqueous solutions, emulsions, suspensions, solutions and/or suspensions reconstituted from non-effervescent granules, and effervescent preparations reconstituted from effervescent granules, containing suitable solvents, preservatives, emulsifying agents, suspending agents, diluents, sweeteners, melting agents, coloring agents and flavoring agents.

The pharmaceutically-acceptable carriers suitable for the preparation of unit dosage forms for peroral administration is well-known in the art. Tablets typically comprise conventional pharmaceutically-compatible adjuvants as inert diluents, such as calcium carbonate, sodium carbonate, mannitol, lactose and cellulose; binders such as starch, gelatin and sucrose; disintegrants such as starch, alginic acid and croscarmelose; lubricants such as magnesium stearate, stearic acid and talc. Glidants such as silicon dioxide can be used to improve flow characteristics of the powder mixture. Coloring agents, such as the FD&C dyes, can be added for appearance. Sweeteners and flavoring agents, such as aspartame, saccharin, menthol, peppermint, and fruit flavors, are useful adjuvants for chewable tablets. Capsules typically comprise one or more solid diluents disclosed above. The selection of carrier components depends on secondary considerations like taste, cost, and shelf stability, which are not critical, and can be readily made by a person skilled in the art.

Peroral compositions also include liquid solutions, emulsions, suspensions, and the like. The pharmaceutically-acceptable carriers suitable for preparation of such compositions are well known in the art. Typical components of carriers for syrups, elixirs, emulsions and suspensions include ethanol, glycerol, propylene glycol, polyethylene glycol, liquid sucrose, sorbitol and water. For a suspension, typical suspending agents include methyl cellulose, sodium carboxymethyl cellulose, AVICEL RC-591, tragacanth and sodium alginate; typical wetting agents include lecithin and polysorbate 80; and typical preservatives include methyl paraben and sodium benzoate. Peroral liquid compositions may also contain one or more components such as sweeteners, flavoring agents and colorants disclosed above.

Such compositions may also be coated by conventional methods, typically with pH or time-dependent coatings, such that the subject composition is released in the gastrointestinal tract in the vicinity of the desired topical application, or at various times to extend the desired action. Such dosage forms typically include, but are not limited to, one or more of cellulose acetate phthalate, polyvinylacetate phthalate, hydroxypropyl methyl cellulose phthalate, ethyl cellulose, Eudragit coatings, waxes and shellac.

Compositions described herein may optionally include other drug actives.

Other compositions useful for attaining systemic delivery of the subject compounds include sublingual, buccal and nasal dosage forms. Such compositions typically comprise one or more of soluble filler substances such as sucrose, sorbitol and mannitol; and binders such as acacia, microcrystalline cellulose, carboxymethyl cellulose and hydroxypropyl methyl cellulose. Glidants, lubricants, sweeteners, colorants, antioxidants and flavoring agents disclosed above may also be included.

A liquid composition, which is formulated for topical ophthalmic use, is formulated such that it can be administered topically to the eye. The comfort may be maximized as much as possible, although sometimes formulation considerations (e.g. drug stability) may necessitate less than optimal comfort. In the case that comfort cannot be maximized, the liquid may be formulated such that the liquid is tolerable to the patient for topical ophthalmic use. Additionally, an ophthalmically acceptable liquid may either be packaged for single use or contain a preservative to prevent contamination over multiple uses.

For ophthalmic application, solutions or medicaments are often prepared using a physiological saline solution as a major vehicle. Ophthalmic solutions may preferably be maintained at a comfortable pH with an appropriate buffer system. The formulations may also contain conventional, pharmaceutically acceptable preservatives, stabilizers and surfactants.

Preservatives that may be used in the pharmaceutical compositions disclosed herein include, but are not limited to, benzalkonium chloride, PHMB, chlorobutanol, thimerosal, phenylmercuric, acetate and phenylmercuric nitrate. A useful surfactant is, for example, Tween 80. Likewise, various useful vehicles may be used in the ophthalmic preparations disclosed herein. These vehicles include, but are not limited to, polyvinyl alcohol, povidone, hydroxypropyl methyl cellulose, poloxamers, carboxymethyl cellulose, hydroxyethyl cellulose and purified water.

Tonicity adjustors may be added as needed or convenient. They include, but are not limited to, salts, particularly sodium chloride, potassium chloride, mannitol and glycerin, or any other suitable ophthalmically acceptable tonicity adjustor.

Various buffers and means for adjusting pH may be used so long as the resulting preparation is ophthalmically acceptable. For many compositions, the pH will be between 4 and 9. Accordingly, buffers include acetate buffers, citrate buffers, phosphate buffers and borate buffers. Acids or bases may be used to adjust the pH of these formulations as needed.

Ophthalmically acceptable antioxidants include, but are not limited to, sodium metabisulfite, sodium thiosulfate, acetylcysteine, butylated hydroxyanisole and butylated hydroxytoluene.

Other excipient components, which may be included in the ophthalmic preparations, are chelating agents. A useful chelating agent is edetate disodium (EDTA), although other chelating agents may also be used in place or in conjunction with it.

For topical use, creams, ointments, gels, solutions or suspensions, etc., containing the composition disclosed herein are employed. Topical formulations may generally be comprised of a pharmaceutical carrier, co-solvent, emulsifier, penetration enhancer, preservative system, and emollient.

For intravenous administration, the compositions described herein may be dissolved or dispersed in a pharmaceutically acceptable diluent, such as a saline or dextrose solution. Suitable excipients may be included to achieve the desired pH, including but not limited to NaOH, sodium carbonate, sodium acetate, HCl, and citric acid. In various embodiments, the pH of the final composition ranges from 2 to 8, or preferably from 4 to 7. Antioxidant excipients may include sodium bisulfite, acetone sodium bisulfite, sodium formaldehyde, sulfoxylate, thiourea, and EDTA. Other non-limiting examples of suitable excipients found in the final intravenous composition may include sodium or potassium phosphates, citric acid, tartaric acid, gelatin, and carbohydrates such as dextrose, mannitol, and dextran. Further acceptable excipients are described in Powell, et al., Compendium of Excipients for Parenteral Formulations, PDA J Pharm Sci and Tech 1998, 52 238-311 and Nema et al., Excipients and Their Role in Approved Injectable Products: Current Usage and Future Directions, PDA J Pharm Sci and Tech 2011, 65 287-332. Antimicrobial agents may also be included to achieve a bacteriostatic or fungistatic solution, including but not limited to phenylmercuric nitrate, thimerosal, benzethonium chloride, benzalkonium chloride, phenol, cresol, and chlorobutanol.

The compositions for intravenous administration may be provided to caregivers in the form of one or more solids that are reconstituted with a suitable diluent such as sterile water, saline or dextrose in water shortly prior to administration. In other embodiments, the compositions are provided in solution ready to administer parenterally. In still other embodiments, the compositions are provided in a solution that is further diluted prior to administration. In embodiments that include administering a combination of a compound described herein and another agent, the combination may be provided to caregivers as a mixture, or the caregivers may mix the two agents prior to administration, or the two agents may be administered separately.

The actual dose of the active compounds described herein depends on the specific compound, and on the condition to be treated; the selection of the appropriate dose is well within the knowledge of the skilled artisan. In some embodiments, a single dose of Plinabulin may be from about 5 mg/m² to about 150 mg/m² of body surface area, from about 5 mg/m² to about 100 mg/m² of body surface area, from about 10 mg/m² to about 100 mg/m² of body surface area, from about 10 mg/m² to about 80 mg/m² of body surface area, from about 10 mg/m² to about 50 mg/m² of body surface area, from about 10 mg/m² to about 40 mg/m² of body surface area, from about 10 mg/m² to about 30 mg/m² of body surface area, from about 13.5 mg/m² to about 100 mg/m² of body surface area, from about 13.5 mg/m² to about 80 mg/m² of body surface area, from about 13.5 mg/m² to about 50 mg/m² of body surface area, from about 13.5 mg/m² to about 40 mg/m² of body surface area, from about 13.5 mg/m² to about 30 mg/m² of body surface area, from about 15 mg/m² to about 80 mg/m² of body surface area, from about 15 mg/m² to about 50 mg/m² of body surface area, or from about 15 mg/m² to about 30 mg/m² of body surface area. In some embodiments, a single dose of Plinabulin may be from about 13.5 mg/m² to about 30 mg/m² of body surface area. In some embodiments, a single dose of Plinabulin may be about 2 mg/m², about 5 mg/m², about 10 mg/m², about 12.5 mg/m², about 13.5 mg/m², about 15 mg/m², about 17.5 mg/m², about 20 mg/m², about 22.5 mg/m², about 25 mg/m². about 27.5 mg/m², about 30 mg/m², about 40 mg/m², about 50 mg/m², about 60 mg/m², about 70 mg/m², about 80 mg/m², about 90 mg/m², or about 100 mg/m², of body surface area. In some embodiments, a single dose of Plinabulin may be from about 2 mg/m² to about 40 mg/m² or from 5 mg/m² to about 35 mg/m².

In some embodiments, a single dose of Plinabulin may be from 5 mg to 300 mg, about 5 mg to about 300 mg, from 5 mg to 200 mg, from about 5 mg to about 200 mg, from 7.5 mg to 200 mg, from about 7.5 mg to about 200 mg, from 10 mg to 100 mg, from about 10 mg to about 100 mg, from about 15 mg to about 100 mg, from about 20 mg to about 100 mg, from about 30 mg to about 100 mg, from about 40 mg to about 100 mg, from about 10 mg to about 80 mg, from about 15 mg to about 80 mg, from about 20 mg to about 80 mg, from about 30 mg to about 80 mg, from about 40 mg to about 80 mg, from about 10 mg to about 60 mg, from about 15 mg to about 60 mg, from about 20 mg to about 60 mg, from about 30 mg to about 60 mg, or from about 40 mg to about 60 mg, In some embodiments, a single dose of Plinabulin may be from 20 mg to 60 mg, from 27 mg to 60 mg, from 20 mg to 45 mg, or from 27 mg to 45 mg. In some embodiments, a single dose of Plinabulin may be from about 20 mg to about 60 mg, from about 27 mg to about 60 mg, from about 20 mg to about 45 mg, or from about 27 mg to about 45 mg. In some embodiments, a single dose of Plinabulin may be, or may be about, 5 mg, 10 mg, 12.5 mg, 13.5 mg, 15 mg, 17.5 mg, 20 mg, 22.5 mg, 25 mg. 27 mg, 30 mg, 40 mg, 50 mg, 60 mg, 70 mg, 80 mg, 90 mg, 100 mg, 125 mg, 150mg, or 200 mg.

The administration period can be a multi-week treatment cycle as long as the tumor remains under control and the regimen is clinically tolerated. In some embodiments, a single dosage of Plinabulin can be administered once a week, and preferably once on each of day 1 and day 8 of a three-week (21 day) treatment cycle. In some embodiments, a single dosage of Plinabulin can be administered once a week, twice a week, three times per week, four times per week, five times per week, six times per week, or daily during a one-week, two-week, three-week, four-week, or five-week treatment cycle. The administration can be on the same or different day of each week in the treatment cycle.

### Methods of Treatment

The invention relates to plinabulin, or a pharmaceutically acceptable salt thereof, for use in combination with osimertinib for use in a method of treating a cancer or tumor characterized by expression of a mutant form of an epidermal growth factor receptor (EGFR) protein, wherein the mutant form of the EGFR protein comprises an amino acid substitution T790M. In some embodiments, the cancer or tumor is selected from squamous cell cancer, non-small cell lung cancer, glioblastoma, epithelian tumors of the head and neck, squamous carcinoma of the lung, hepatocellular carcinoma, colon cancer, endometrial carcinoma, multiple myeloma, and hepatocellular carcinoma. In some embodiments, the cancer or tumor is selected from Metastatic colorectal cancer (CRC); breast cancer; NSCLC (ALK-positive); renal cell carcinoma (RCC); Thyroid cancer; metastatic medullary; NSCLC (ALK-positive after crizotinib resistance); Melanoma with BRAF mutations; NSCLC (ALK-positive or ROS1-positive ); Melanoma and NSCLC with BRAF mutations; pancreatic cancer; breast cancer(ER2-negative); progressive neuroendocrine tumors of pancreatic origin (PNET) (unresectable, locally advanced or metastatic); renal angiomyolipoma; subependymal giant cell astrocytoma; Mantle cell lymphoma; Chronic lymphocytic leukemia (CLL); Waldenstrom's macro-globulinemia; Philadelphia chromosome positive chronic myeloid leukemia (Ph+ CML); (Ph+ CML) in blast crisis (BC); (Ph+ CML) in accelerated phase (AP); (Ph+ CML) in chronic phase (CP); Philadelphia chromosome positive acute lymphoblastic leukemia (Ph+ ALL); myelodysplastic/myeloproliferative diseases (MDS/MPD) associated with PDGFR (platelet-derived growth factor receptor) gene rearrangements; aggressive systemic mastocytosis (ASM) without the D816V c-Kit mutation; hypereosinophilic syndrome (HES) and/or chronic eosinophilic leukemia (CEL) who have the FIP1L1-PDGFRα fusion kinase (mutational analysis or FISH demonstration of CHIC2 allele deletion); unresectable, recurrent and/or metastatic dermatofibrosarcoma protuberans (DFSP); metastatic malignant gastrointestinal stromal tumors (GIST); MDS/MDP; Acute myeloid leukemia; mastocytosis; mast cell leukemia; HER+ breast cancer; idiopathic pulmonary fibrosis (IPF); breast cancer (ER+ and HER2+); soft tissue sarcomas; myelofibrosis; polycythemia vera; Renal transplant; lymphangio-leiomyomatosis; Thyroid cancer, differentiated; hepatocellular carcinoma; pancreatic neuroendocrine tumors; RCC, advanced; Melanoma; Thyroid cancer, medullary; multiple myeloma, or any combinations thereof. In some embodiments the cancer is melanoma with the BRAFV600E mutation. In some embodiments, the cancer is NSCLC. In some embodiments, the cancer is receptor (EGFR) T790M mutation positive non-small cell lung cancer (NSCLC). In some embodiments, the cancer is multiple myeloma.

According to the invention, the treatment described above or elsewhere herein is for patients with metastatic epidermal growth factor receptor (EGFR) T790M mutation positive non-small cell lung cancer (NSCLC), as detected by an FDA approved test, who have progressed on or after EGFR TKI therapies, including but are not limited to gefitinib, erlotinib, and/or osimertinib.

Some embodiments relate to plinabulin, or a pharmaceutically acceptable salt thereof, for use in combination with osimertinib for use in a method of inhibiting proliferation of a cell having an epidermal growth factor receptor (EGFR) mutation or a cell expressing a mutant form of an EGFR protein, wherein the mutant form of the EGFR protein comprises an amino acid substitution T790M. In some embodiments, the cell is a cancer cell. In some embodiments, the step of contacting comprises administering Plinabulin to a subject having the cell. In some embodiments, the step of contacting comprises administering an effective amount of Plinabulin to the subject having the cell.

Some embodiments relate to plinabulin, or a pharmaceutically acceptable salt thereof, for use in combination with osimertinib for use in a method of inhibiting progression of a cancer characterized by expression of a mutant form of an EGFR protein in a subject, wherein the mutant form of the EGFR protein comprises an amino acid substitution T790M. In some embodiments, the step of administering comprises administering an effective amount of plinabulin to the subject in need thereof.

In some embodiments described herein, the mutant form of the EGFR protein comprises mutation at one or more positions selected from the group consisting of G719, L858, L861, T790, T854, D761, exon 19, and exon 20. In some embodiments, the mutation in exon 19 comprises a deletion mutation in exon 19. In some embodiments, the mutation in exon 20 comprises an insertion mutation in exon 20. In some embodiments, the mutant form of the EGFR protein comprises one or more amino acid substitutions selected from one or more positions consisting of G719S, G719C, G719A, L858R, L861Q, T854A, and D761Y. According to the invention, the mutant form of the EGFR protein comprises an amino acid substitution T790M.

In some embodiments, the method described above or elsewhere herein comprises determining whether the subject has an EGFR mutation. In some embodiments, the method described above or elsewhere herein comprises identifying whether the subject has a metastatic EGFR T790M mutation-positive tumor.

According to the invention, the method described herein comprises administering one or more additional active agents, wherein the additional active agent is an additional chemotherapeutic agent, wherein the additional chemotherapeutic agent is osimertinib. In some embodiments, the additional chemotherapeutic agent is a tyrosine kinase inhibitor. In some embodiments, the additional therapy is radiotherapy.

In some embodiments, the tyrosine kinase inhibitor (e.g., osimertinib) is administered prior to, concurrently, or after the administration of plinabulin. In some embodiments, the tyrosine kinase inhibitor is administered prior to the administration of plinabulin. In some embodiments, the tyrosine kinase inhibitor is administered after the administration of plinabulin. In some embodiments, the tyrosine kinase inhibitor is administered concurrently with the administration of plinabulin. In some embodiments, the tyrosine kinase inhibitor is selected from the group consisting of Acalabrutinib (ACP-196), Afatinib (BIBW 2992, Tovok, OWN, PDB ID: 4G5J with EGFR), Alectinib (CH5424802, Alecensa, EMH, PDB ID: 3AOX with ALK), Axitinib (AG-013736, Inlyta, AXI, PDB ID: 4AG8 with VEGFR2), Bosutinib (SKI-606, BOSULIF, DB8, PDB ID: 3UE4 with Abl), Brigatinib (AP 26113, Alunbrig, PDB ID: 5J7H with ALK), Cabozantinib (XL-184, BMS-907351, Cometriq, no X-ray structure), Ceritinib (LDK378, Zykadia, 4MK, PDB ID: 4MKC with ALK), Cobimetinib (GDC-0973, EUI, Cotellic. PDB ID: 4AN2 with MEK1), Crizotinib (PF 2341066, VGH, Xalkori, PDB: 2XB2 for ALK; 3ZBF for ROS; 2WGJ with MET), Dabrafenib (GSK2118436, Tafinlar, P06, PDB ID: 5CSW with B-Raf), Dasatinib (BMS-354825, Sprycel, 1N1. PDB ID: 2GQG with Abl), Erlotinib (CP-358774, OSI-774, Tarceva, AQ4, PDB ID: 4HJO and 1M17 with EGFR), Everolimus (RAD001, Afinitor), Gefitinib (ZD1839, Iressa, IRE, PDB ID: 4WKQ and 2ITY with EGFR), Ibrutinib (PCI-32765, Imbruvica, PDB ID: 5P9J with BTK1), Imatinib (STI571, Gleevec, STI, PDB ID: 2HYY and 1IEP with Abl), Lapatinib (GW572016, Tykerb, FMM, PDB ID: 1XKK with EGFR), Lenvatinib (AK175809, Lenvima, LEV, PDB ID: 3WZD with VEGFR2), Midostaurin (PKC412, CPG 41251, Rydapt), Neratinib (HKI-272, PDB ID: 2JIV with EGFR), Nilotinib (AMN107, Tasigna, NILK, PDB ID: 3CS9 with Abl), Nintedanib, BIBF-1120, Vargatef, XIN, PDB ID: 3C7Q with VEGFR2), Osimertinib (AZD-9292, Tagrisso), Palbociclib (PD-0332991, Ibrance, LQQ, PDB ID: 5L2I), Pazopanib (GW786034, Votrient), Ponatinib (AP 24534, Iclusig, OLI, PDB ID: 4U0I with Kit, 3OXZ with Abl, 1UWH with B-Raf), Regorafenib (BAY 73-4506, Stivarga), Ribociclib (LEE011, Kisqali, PDB ID: 5L2T), Ruxolitinib (INCB-018424, Jakafi, RXT, PDB ID: 4U5J with Src), Sirolimus (Rapamycin), Sorafenib (BAY 43-9006, Nexavar, BAX, PDB ID: 4ASD with VEGFR2), Sunitinib (SU-11248, Sutent, B49, PDB ID: 4AGD with VEGFR2), Temsirolimus (CCI-779, Torisel), Tofacitinib (CP-690550, Tasocitinib, MI1, PDB:ID 3EYG with JAK1, 3LXK with JAK3), Trametinib (Mekinist), Vandetanib (ZD6474, Zactima, ZD6, PDB ID:2IVU with RET), Vemurafenib (PLX-4032, Zelboraf, O32, PDB ID: 4RZV and 3OG7 with B-Raf), and any combinations thereof. According to the invention, the tyrosine kinase inhibitor is osimertinib. In some embodiments, the tyrosine kinase inhibitor (e.g., osimertinib), as described above or elsewhere herein, is administered orally. In some embodiments, the osimertinib is administered at a dose of, or at a dose of about, 80 mg per day. In some embodiments, the osimertinib is administered at a dose of, or at a dose of about, 10 mg, 20 mg, 30 mg, 40 mg, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, or 200 mg per day. In some embodiments, the osimertinib is administered in the range of 1 mg to 200 mg per day, about 1 mg to about 200 mg per day, 10 mg to 200 mg per day, about 10 mg to about 200 mg per day, 20 mg to 160 mg per day, about 20 mg to about 160 mg per day, 40 mg to 160 mg per day, about 40 mg to about 160 mg per day, 40 mg to 140 mg per day, or about 40 mg to about 140 mg per day. In some embodiments, the osimertinib is administered in the range of 60 mg to 100 mg, or about 60 mg to about 100 mg, per day. In some embodiments, the osimertinib is administered at an amount that is greater than, or greater than about, 5 mg, 10 mg, 20 mg, 30 mg, 40 mg, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, or 200 mg per day. In some embodiments, the osimertinib is administered at an amount that is less than, or less than about, 5 mg, 10 mg, 20 mg, 20 mg, 40 mg, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, or 200 mg per day.

According to the invention, the plinabulin is administered in combination with the tyrosine kinase inhibitor, osimertinib. In some embodiments, the plinabulin is administered parenterally. In some embodiments, the plinabulin is administered intramuscularly. In some embodiments, the plinabulin is administered intravenously. In some embodiments, the plinabulin is administered at a dose in the range of 2.5 to 35 mg/mm², or about 2.5 to about 35 mg/mm². In some embodiments, the plinabulin is administered at a dose in the range of 2.5 to 40 mg/mm², about 2.5 to about 40 mg/mm², 5 to 40 mg/mm², or about 5 to about 40 mg/mm². In some embodiments, the plinabulin is administered at a dose in the range of 2.5 to 37.5 mg/mm², about 2.5 to about 37.5 mg/mm², 5 to 35 mg/mm², or about 5 to about 35 mg/mm². In some embodiments, the plinabulin and the osimertinib are administered on a different schedule. In some embodiments, the plinabulin and the osimertinib are administered on the same schedule. In some embodiments, the plinabulin is administered at a dose in the range of, or at a dose in the range of about, 1-50 mg/m² of the body surface area. In some embodiments, the plinabulin is administered at a dose in the range of, or in the range of about, 1-2, 1-3, 1-4, 1-5, 1-6, 1-7, 1-8, 1-9, 1-10, 1-11, 1-12, 1-13, 1-13.75, 1-14, 1-15, 1-16, 1-17, 1-18, 1-19, 1-20, 1-22.5, 1-25, 1-27.5, 1-30, 1.5-2, 1.5-3, 1.5-4, 1.5-5, 1.5-6, 1.5-7, 1.5-8, 1.5-9, 1.5-10, 1.5-11, 1.5-12, 1.5-13, 1.5-13.75, 1.5-14, 1.5-15, 1.5-16, 1.5-17, 1.5-18, 1.5-19, 1.5-20, 1.5-22.5, 1.5-25, 1.5-27.5, 1.5-30, 2.5-2, 2.5-3, 2.5-4, 2.5-5, 2.5-6, 2.5-7, 2.5-8, 2.5-9, 2.5-10, 2.5-11, 2.5-12, 2.5-13, 2.5-13.75, 2.5-14, 2.5-15, 2.5-16, 2.5-17, 2.5-18, 2.5-19, 2.5-20, 2.5-22.5, 2.5-25, 2.5-27.5, 2.5-30, 2.5-35, 2.5-40, 2.5-20, 3-4, 3-5, 3-6, 3-7, 3-8, 3-9, 3-10, 3-11, 3-12, 3-13, 3-13.75, 3-14, 3-15, 3-16, 3-17, 3-18, 3-19, 3-20, 3-22.5, 3-25, 3-27.5, 3-30, 3-40, 3-50, 3.5- 6.5, 3.5-13.75, 3.5-15, 2.5-17.5, 4-5, 4-6, 4-7, 4-8, 4-9, 4-10, 4-11, 4-12, 4-13, 4-13.75, 4-14, 4-15, 4-16, 4-17, 4-18, 4-19, 4-20, 4-22.5, 4-25, 4-27.5, 4-30, 5-6, 5-7, 5-8, 5-9, 5-10, 5-11, 5-12, 5-13, 5-13.75, 5-14, 5-15, 5-16, 5-17, 5-18, 5-19, 5-20, 5-22.5, 5-25, 5-27.5, 5-30, 5-40, 5-50, 6-7, 6-8, 6-9, 6-10, 6-11, 6-12, 6-13, 6-13.75, 6-14, 6-15, 6-16, 6-17, 6-18, 6-19, 6-20, 6-22.5, 6-25, 6-27.5, 6-30, 6-40, 6-50, 7-8, 7-9, 7-10, 7-11, 7-12, 7-13, 7-13.75, 7-14, 7-15, 7-16, 7-17, 7-18, 7-19, 7-20, 7-22.5, 7-25, 7-27.5, 7-30, 7-40, 7-50, 7.5-12.5, 7.5-13.5, 7.5-15, 8-9, 8-10, 8-11, 8-12, 8-13, 8-13.75, 8-14, 8-15, 8-16, 8-17, 8-18, 8-19, 8-20, 8-22.5, 8-25, 8-27.5, 8-30, 8-40, 8-50, 9-10, 9-11, 9-12, 9-13, 9-13.75, 9-14, 9-15, 9-16, 9-17, 9-18, 9-19, 9-20, 9-22.5, 9-25, 9-27.5, 9-30, 9-40, 9-50, 10-11, 10-12, 10-13, 10-13.75, 10-14, 10-15, 10-16, 10-17, 10-18, 10-19, 10-20, 10-22.5, 10-25, 10-27.5, 10-30, 10-40, 10-50, 11.5-15.5, 12.5-14.5, 7.5-22.5, 8.5-32.5, 9.5-15.5, 15.5-24.5, 5-35, 17.5-22.5, 22.5-32.5, 25-35, 25.5-24.5, 27.5-32.5, 2-20, t 2.5-22.5, or 9.5-21.5 mg/m², of the body surface area. In some embodiments, the plinabulin is administered at a dose of, or at a dose of about, 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 32, 33, 34, 35, 36, 37, 38, 39, 40, 50 mg/m² of the body surface area. In some embodiments, the plinabulin is administered at a dose less than, or at a dose less than about, 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 32, 33, 34, 35, 36, 37, 38, 39, 40, 45, 50 mg/m² of the body surface area. In some embodiments, the plinabulin is administered at a dose greater than, or at a dose greater than about, 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5, 30, 30.5, 31, 32, 33, 34, 35, 36, 37, 38, 39, 40, 41, 42, 43, 44, 45, 46, 47, 48, 49, 50 mg/m² of the body surface area. In some embodiments, the plinabulin dose is in the range of, or in the range of about, 5 mg - 300 mg, 5 mg -200 mg, 7.5 mg - 200 mg, 10 mg - 100 mg, 15 mg - 100 mg, 20 mg - 100 mg, 30 mg - 100 mg, 40 mg - 100 mg, 10 mg - 80 mg, 15 mg - 80 mg, 20 mg - 80 mg, 30 mg - 80 mg, 40 mg - 80 mg, 10 mg - 60 mg, 15 mg - 60 mg, 20 mg - 60 mg, 30 mg - 60 mg, or about 40 mg - 60 mg. In some embodiments, the plinabulin administered is in the range of, or in the range of about, 20 mg - 60 mg, 27 mg - 60 mg, 20 mg - 45 mg, or 27 mg - 45 mg. In some embodiments, the plinabulin administered is in the range of, or in the range of about, 5 mg-7.5 mg, 5 mg-9 mg, 5 mg-10 mg, 5 mg-12mg, 5mg-14mg, 5mg-15 mg, 5 mg-16 mg, 5 mg-18 mg, 5 mg-20 mg, 5 mg-22 mg, 5 mg-24 mg, 5 mg-26 mg, 5 mg-28mg, 5mg-30mg, 5mg-32mg, 5mg-34mg, 5mg-36mg, 5mg-38mg, 5mg-40mg, 5mg-42mg, 5mg-44mg, 5mg-46mg, 5mg-48mg, 5mg-50mg, 5mg-52mg, 5mg-54mg, 5mg-56mg, 5mg-58mg, 5mg-60mg, 7 mg-7.7 mg, 7 mg-9 mg, 7 mg-10 mg, 7 mg-12mg, 7mg-14mg, 7mg-15 mg, 7 mg-16 mg, 7 mg-18 mg, 7 mg-20 mg, 7 mg-22 mg, 7 mg-24 mg, 7 mg-26 mg, 7 mg-28mg, 7mg-30mg, 7mg-32mg, 7mg-34mg, 7mg-36mg, 7mg-38mg, 7mg-40mg, 7mg-42mg, 7mg-44mg, 7mg-46mg, 7mg-48mg, 7mg-50mg, 7mg-52mg, 7mg-54mg, 7mg-56mg, 7mg-58mg, 7mg-60mg, 9 mg-10 mg, 9 mg-12mg, 9mg-14mg, 9mg-15 mg, 9 mg-16 mg, 9 mg-18 mg, 9 mg-20 mg, 9 mg-22 mg, 9 mg-24 mg, 9 mg-26 mg, 9 mg-28mg, 9mg-30mg, 9mg-32mg, 9mg-34mg, 9mg-36mg, 9mg-38mg, 9mg-40mg, 9mg-42mg, 9mg-44mg, 9mg-46mg, 9mg-48mg, 9mg-50mg, 9mg-52mg, 9mg-54mg, 9mg-56mg, 9mg-58mg, 9mg-60mg, 10 mg-12mg, 10mg-14mg, 10mg-15 mg, 10 mg-16 mg, 10 mg-18 mg, 10 mg-20 mg, 10 mg-22 mg, 10 mg-24 mg, 10 mg-26 mg, 10 mg-28mg, 10mg-30mg, 10mg-32mg, 10mg-34mg, 10mg-36mg, 10mg-38mg, 10mg-40mg, 10mg-42mg, 10mg-44mg, 10mg-46mg, 10mg-48mg, 10mg-50mg, 10mg-52mg, 10mg-54mg, 10mg-56mg, 10mg-58mg, 10mg-60mg, 12mg-14mg, 12mg-15 mg, 12 mg-16 mg, 12 mg-18 mg, 12 mg-20 mg, 12 mg-22 mg, 12 mg-24 mg, 12 mg-26 mg, 12 mg-28mg, 12mg-30mg, 12mg-32mg, 12mg-34mg, 12mg-36mg, 12mg-38mg, 12mg-40mg, 12mg-42mg, 12mg-44mg, 12mg-46mg, 12mg-48mg, 12mg-50mg, 12mg-52mg, 12mg-54mg, 12mg-56mg, 12mg-58mg, 12mg-60mg, 15 mg-16 mg, 15 mg-18 mg, 15 mg-20 mg, 15 mg-22 mg, 15 mg-24 mg, 15 mg-26 mg, 15 mg-28mg, 15mg-30mg, 15mg-32mg, 15mg-34mg, 15mg-36mg, 15mg-38mg, 15mg-40mg, 15mg-42mg, 15mg-44mg, 15mg-46mg, 15mg-48mg, 15mg-50mg, 15mg-52mg, 15mg-54mg, 15mg-56mg, 15mg-58mg, 15mg-60mg, 17 mg-18 mg, 17 mg-20 mg, 17 mg-22 mg, 17 mg-24 mg, 17 mg-26 mg, 17 mg-28mg, 17mg-30mg, 17mg-32mg, 17mg-34mg, 17mg-36mg, 17mg-38mg, 17mg-40mg, 17mg-42mg, 17mg-44mg, 17mg-46mg, 17mg-48mg, 17mg-50mg, 17mg-52mg, 17mg-54mg, 17mg-56mg, 17mg-58mg, 17mg-60mg, 20 mg-22 mg, 20 mg-24 mg, 20 mg-26 mg, 20 mg-28mg, 20mg-30mg, 20mg-32mg, 20mg-34mg, 20mg-36mg, 20mg-38mg, 20mg-40mg, 20mg-42mg, 20mg-44mg, 20mg-46mg, 20mg-48mg, 20mg-50mg, 20mg-52mg, 20mg-54mg, 20mg-56mg, 20mg-58mg, 20mg-60mg, 22 mg-24 mg, 22 mg-26 mg, 22 mg-28mg, 22mg-30mg, 22mg-32mg, 22mg-34mg, 22mg-36mg, 22mg-38mg, 22mg-40mg, 22mg-42mg, 22mg-44mg, 22mg-46mg, 22mg-48mg, 22mg-50mg, 22mg-52mg, 22mg-54mg, 22mg-56mg, 22mg-58mg, 22mg-60mg, 25 mg-26 mg, 25 mg-28mg, 25mg-30mg, 25mg-32mg. 25mg-34mg, 25mg-36mg, 25mg-38mg, 25mg-40mg, 25mg-42mg, 25mg-44mg, 25mg-46mg, 25mg-48mg, 25mg-50mg, 25mg-52mg, 25mg-54mg, 25mg-56mg, 25mg-58mg, 25mg-60mg, 27 mg-28mg, 27mg-30mg, 27mg-32mg, 27mg-34mg, 27mg-36mg, 27mg-38mg, 27mg-40mg, 27mg-42mg, 27mg-44mg, 27mg-46mg, 27mg-48mg, 27mg-50mg, 27mg-52mg, 27mg-54mg, 27mg-56mg, 27mg-58mg, 27mg-60mg, 30mg-32mg, 30mg-34mg, 30mg-36mg, 30mg-38mg, 30mg-40mg, 30mg-42mg, 30mg-44mg, 30mg-46mg, 30mg-48mg, 30mg-50mg, 30mg-52mg, 30mg-54mg, 30mg-56mg, 30mg-58mg, 30mg-60mg, 33mg-34mg, 33mg-36mg, 33mg-38mg, 33mg-40mg, 33mg-42mg, 33mg-44mg, 33mg-46mg, 33mg-48mg, 33mg-50mg, 33mg-52mg, 33mg-54mg, 33mg-56mg, 33mg-58mg, 33mg-60mg, 36mg-38mg, 36mg-40mg, 36mg-42mg, 36mg-44mg, 36mg-46mg, 36mg-48mg, 36mg-50mg, 36mg-52mg, 36mg-54mg, 36mg-56mg, 36mg-58mg, 36mg-60mg, 40mg-42mg, 40mg-44mg, 40mg-46mg, 40mg-48mg, 40mg-50mg, 40mg-52mg, 40mg-54mg, 40mg-56mg, 40mg-58mg, 40mg-60mg, 43mg-46mg, 43mg-48mg, 43mg-50mg, 43mg-52mg, 43mg-54mg, 43mg-56mg, 43mg-58mg, 42mg-60mg, 45mg-48mg, 45mg-50mg, 45mg-52mg, 45mg-54mg, 45mg-56mg, 45mg-58mg, 45mg-60mg, 48mg-50mg, 48mg-52mg, 48mg-54mg, 48mg-56mg, 48mg-58mg, 48mg-60mg, 50mg-52mg, 50mg-54mg, 50mg-56mg, 50mg-58mg, 50mg-60mg, 52mg-54mg, 52mg-56mg, 52mg-58mg, or 52mg-60mg. In some embodiments, the plinabulin dose is greater than, or greater than about, 5 mg, 10 mg, 12.5 mg, 13.5 mg, 15 mg, 17.5 mg, 20 mg, 22.5 mg, 25 mg, 27 mg. 30 mg, 40 mg, 50 mg, 60 mg, 70 mg, 80 mg, 90 mg, 100 mg, 125 mg, 150mg, or 200 mg. In some embodiments, the plinabulin dose is less than, or less than about, 5 mg, 10 mg, 12.5 mg, 13.5 mg, 15 mg, 17.5 mg, 20 mg, 22.5 mg, 25 mg, 27 mg, 30 mg, 40 mg, 50 mg, 60 mg, 70 mg, 80 mg, 90 mg, 100 mg, 125 mg, 150mg, or 200 mg. In some embodiments, the plinabulin is administered in combination with an epidermal growth factor receptor (EGFR) tyrosine kinase inhibitor. According to the invention, the plinabulin is administered in combination with osimertinib. The administration period can be a multi-week treatment cycle as long as the tumor remains under control and the regimen is clinically tolerated. In some embodiments, osimertinib and plinabulin can be administered once every three weeks. In some embodiments, osimertinib and plinabulin can be administered once every week, once every two weeks, once every three weeks, once every four weeks, once evert five weeks, or once every six weeks. In some embodiments, osimertinib and Plinabulin can be administered once a week, and preferably once on each of day 1 and day 8 of a three-week (21 day) treatment cycle. In some embodiments, osimertinib and Plinabulin can be administered once a week, twice a week, three times per week, four times per week, five times per week, six times per week, or daily during a one-week, two-week, three-week, four-week, or five-week treatment cycle. The administration can be on the same or different day of each week in the treatment cycle. In some embodiments, the plinabulin is administered prior to the osimertinib administration. In some embodiments, the plinabulin is administered concurrently with the osimertinib administration. In some embodiments, the plinabulin is administered after the osimertinib administration. In some embodiments, the plinabulin is administered after the tyrosine kinase inhibitor (e.g., osimertinib) administration. In some embodiments, the plinabulin is administered, or administered about, 1 min, 5min, 10 min, 15 min, 20 min, 25 min, 30 min, 1h, 1.5h, 2h, 2.5h, 3h, 4h, 5h, 6h, 7h, 8h, 9h, 10h, 11h, or 12h after the administration of the tyrosine kinase inhibitor. In some embodiments, the plinabulin is administered in less than, or in less than about, 1 min, 5min, 10 min, 15 min, 20 min, 25 min, 30 min, 1h, 1.5h, 2h, 2.5h, 3h, 4h, 5h, 6h, 7h, 8h, 9h, 10h, 11h, 12h, 13h, 14h, 15h. 16h, 17h, 18h, 19h, 20h, 21h, 22h, 23h, or 24h after the administration of the tyrosine kinase inhibitor. In some embodiments, the plinabulin is administered in more than, or in more than about, 1 min, 5min, 10 min, 15 min, 20 min, 25 min, 30 min, 1h, 1.5h, 2h, 2.5h, 3h, 4h, 5h, 6h, 7h, 8h, 9h, 10h, 11h, 12h, 13h, 14h, 15h, 16h, 17h, 18h, 19h, 20h, 21h, 22h, 23h, or 24h after the administration of the tyrosine kinase inhibitor. In some embodiments, the plinabulin is administered in, or is administered in about, 1min-5min, 1min-10min, 1min-15min, 1min-20min, 1 min-25min, 1 min-30min, 0.25h-0.5h, 0.25-0.75h, 0.25-1h,0.5h-1h, 0.5h-2h, 0.5h-2.5h, 1h-2h, 1h-3h, 1h-5h after the administration of the tyrosine kinase inhibitor.

In some embodiments, when plinabulin is administered prior to the tyrosine kinase inhibitor (e.g., osimertinib) administration, the plinabulin is administered, or is administered about, 1min-5min, 1min-10min, 1min-15min, 1min-20min, 1 min-25min, 1 min-30min, 0.25h-0.5h, 0.25-0.75h, 0.25-1h,0.5h-1h, 0.5h-2h, 0.5h-2.5h, 1h-2h, 1h-3h, 1h-5h before the administration of the tyrosine kinase inhibitor. In some embodiments, the plinabulin is administered, or is administered about, 1 min, 5min, 10 min, 15 min, 20 min, 25 min, 30 min, 1h, 1.5h, 2h, 2.5h, 3h, 4h, 5h, 6h, 7h, 8h, 9h, 10h, 11h, or 12h before the administration of the tyrosine kinase inhibitor. In some embodiments, the plinabulin is administered in less than, or in less than about, 1 min, 5min, 10 min, 15 min, 20 min, 25 min, 30 min, 1h, 1.5h, 2h, 2.5h, 3h, 4h, 5h, 6h, 7h, 8h, 9h, 10h, 11h, 12h, 13h, 14h, 15h, 16h, 17h, 18h, 19h, 20h, 21h, 22h, 23h, or 24h before the administration of the tyrosine kinase inhibitor. In some embodiments, the plinabulin is administered in more than, or in more than about, 1 min, 5min, 10 min, 15 min, 20 min, 25 min, 30 min, 1h, 1.5h, 2h, 2.5h, 3h, 4h, 5h, 6h, 7h, 8h, 9h, 10h, 11h, 12h, 13h, 14h, 15h. 16h, 17h, 18h, 19h, 20h, 21h, 22h, 23h, or 24h before the administration of the tyrosine kinase inhibitor.

In some embodiments, the treatment schedule includes administration of the tyrosine kinase inhibitor (e.g., osimertinib) followed by the administration of plinabulin once every 3 weeks. In some embodiments, the treatment schedule includes administration of the tyrosine kinase inhibitor followed by the administration of plinabulin once every 1 week, 2 weeks, 3 weeks, 4 weeks, 5 weeks, 6 weeks, 7 weeks, or 8 weeks. In some embodiments, the treatment schedule includes administration of the tyrosine kinase inhibitor followed by the administration of plinabulin two times every 1 week, 2 weeks, 3 weeks, 4 weeks, 5 weeks, 6 weeks, 7 weeks, or 8 weeks. In some embodiments, the treatment schedule includes administration of the tyrosine kinase inhibitor followed by the administration of plinabulin once every 1 week in a treatment cycle of 1 week, 2 weeks, 3 weeks, 4 weeks, 5 weeks, 6 weeks, 7 weeks, or 8 weeks. In some embodiments, the treatment schedule includes administration of the tyrosine kinase inhibitor followed by the administration of plinabulin twice every 1 week in a treatment cycle of 1 week, 2 weeks, 3 weeks, 4 weeks, 5 weeks, 6 weeks, 7 weeks, or 8 weeks. In some embodiments, the treatment schedule includes administration of the tyrosine kinase inhibitor followed by the administration of plinabulin on day 1, day 8, and day 15 of a 21-day treatment cycle. The treatment cycle can be repeated as long as the regimen is clinically tolerated. In some embodiments, the treatment cycle for osimertinib and plinabulin is repeated for n times, wherein n is an integer in the range of 2 to 30. In some embodiments, n is 2, 3, 4, 5, 6, 7, 8, 9, or 10. In some embodiments, a new treatment cycle can occur immediately after the completion of the previous treatment cycle. In some embodiments, a new treatment cycle can occur a period of time after the completion of the previous treatment cycle.

### Example

### Example 1

The therapeutic efficacy of Osimertinib and Plinabulin were tested *in vivo.* The two drugs were tested as a single agent and in combination in the treatment of subcutaneous NCI-H1975 Human Lung Cancer Xenograft Model in Balb/c Nude Mice.

The administration of the test articles and the animal numbers in each study group were shown in the following experimental design table 1.

**Table 1. Study Design**

| **Group** | **N** | **Treatment** | **Dose(mg/kg)** | **Dosing Route** | **Schedule** |
|---|---|---|---|---|---|
| 1 | 10 | Vehicle | -- | p.o. | QD x 5/wk, 2 wks Day 1-5, 8-10 |
| 2 | 10 | Tagrisso(osimertinib or AZD-9291) | 2.5mg/kg | p.o. | QD x x5/wk, 2 wks\ Day 1-5, 8-10 |
| 3 | 10 | Plinabulin | 7.5 | i.p. | BIW, 2 wks (Day 1,4,8,11) |
| 4 | 10 | Plinabulin | 2.5 | i.p. | BIW, 2 wks. (Day 1,4,8,11) |
| 5 | 10 | Plinabulin+ Tagrisso(osimertinib or AZD-9291) | Osi. 2.5 mg/kg | Osi oral Plin i.p. | Osi Day 1-5, 8-10 Plin 60 min post Osi on Day 1,4,8,11 |
| | | | Plin 7.5 mg/kg | | |
| 6 | 10 | Plinabulin+ Tagrisso(osimertinib or AZD-9291) | Osi 2.5 mg/kg | Osi oral Plin i.p. | Osi Day 1-5, 8-10 Plin 60 min post Osi Day 1,4,8,11 |
| | | | Plin 2.5 mg/kg | | |

| | | | | | |
|---|---|---|---|---|---|
| *N: animal number; Osi: Osimertinib; Plin: plinabulin Dosing volume: adjust dosing volume based on body weight (10 µl/g). Treatment regimen may be changed per BW loss or other adverse effect according to rules set forth and/or client requests. | | | | | |

Study endpoints: The major endpoints of the study include the followings: Tumor growth inhibition (TGI): TGI% was an indication of antitumor effectiveness, and expressed as: TGI (%) =100 x (1-T/C). T and C were the mean tumor volume (or weight) of the treated and control groups, respectively, on a given day.

The test uses 6-8 weeks female mice (species: Mus Musculus, Strain: Balb/c nude). The average body weight of the mice was 18-22 g. A total of 84 mice (60 plus 40% spare) were used. The animals were housed in individual ventilated cages (up to 5 mice per cage) under the following conditions: Temperature: 20-26°C; Humidity: 40-70%; Light cycle: 7:00 am - 19:00 pm light and 19:00 pm - 7:00 am (next day) darkness; Polysulfone IVC cage: size of 325 mm x 210 mm x 180 mm; Bedding material: corn cob; Diet: Mouse diet, Co60 irradiation sterilized dry granule food. Animals have free access during the entire study period; Water: Reverse osmosis (RO) water, autoclaved before using. Animals have free access to sterile drinking water; Cage identification label: number of animals, sex, strain, receiving date, treatment, study number, group number, and the starting date of the treatment, etc; Animal identification: Animals were marked by ear coding (notch); Adapt housing: the animals were adapted in the facility for at least 7 days.

Cell Culture: The NCI-H1975 tumor cells were maintained in vitro as monolayer culture in MEM medium supplemented with 0.01mM NEAA and 10% heat inactivated fetal bovine serum at 37°C in an atmosphere of 5% CO2 in air. The tumor cells were routinely subcultured twice weekly by trypsin-EDTA treatment. The cells growing in an exponential growth phase were harvested and counted for tumor inoculation.

Tumor Inoculation: Each mouse was inoculated subcutaneously at the right flank region with the NCI-H1975 tumor cells (5×10⁶⁾ in 0.1 ml of PBS for tumor development. The treatments was started when the mean tumor size reaches approximately 150 (100-200) mm3. The test article administration and the animal numbers in each group were shown in the following experiment design Table 2. The date of tumor cell inoculation was denoted as day 0.

Group assignment: Before grouping and treatment, all animals were weighed and the tumor volumes were measured using a caliper. Since the tumor volume can affect the effectiveness of any given treatment, mice were assigned into groups using randomized block design as following:

First, the experimental animals were divided into homogeneous blocks based on their tumor volume. Secondly, each block were randomized to a treatment group. By using randomized block design, it has been ensured that each animal has the same probability to be assigned to any given treatment groups and therefore minimize systematic errors.

The dosing formulation was freshly prepared before each dosing and the volume was adjusted for body weight (Dosing volume = 10 µl/g). The preparation of the dosing material is described in Table 2.

**Table 2. The dosing material preparation**

| Compounds | Preparation | Concentrati on (mg/ml) | Dose (mg/kg) | Physical description | Storage |
|---|---|---|---|---|---|
| Vehicle (1% DMS O+30% PE G 300+69% dd H2O) (p.o.) | Add 34.5 ml dd H2O, then add 15 ml PEG300 and 0.5 ml DMSO into it. Vortex to mix well. | - | - | solution | 4°C |
| Tagrisso (osimertini b or AZD-9291) | Weigh out 2 mg Tagrisso, add 80 µl DMSO, mix well to dissolve completely. Then add 2.4 ml PEG300 and 5.52 ml ddH2O,vortex to mix well. | 0.25 | 2.5 | solution | RT |
| Plinabulin | Weigh out 4.5 mg Plinabulin, add 426 µl Tween 80 and 1.53 ml propylene glycol, mix well to dissolve completely. Then add 4.044 ml D5W, vortex to mix well. | 0.75 | 7.5 | solution | RT |
| Plinabulin | Weigh out 1.5 mg Plinabulin, add 426 µl Tween 80 and 1.53 ml propylene glycol, mix well to dissolve completely. Then add 4.044 ml D5W, vortex to mix well. | 0.25 | 2.5 | solution | RT |

| | | | | | |
|---|---|---|---|---|---|
| Ensure that formulation was mixed immediately before use by gently turning the tube up and down. | | | | | |

Test article administration: The treatment was initiated immediately or one day post grouping per study design (Table 1).

Observation and data collection: After tumor cells inoculation, the animals were checked daily for morbidity and mortality. During routine monitoring, the animals were checked for any effects of tumor growth and treatments on normal behavior such as mobility, visual estimation of food and water consumption, body weight gain/loss (body weights were measured twice per week), eye/hair matting and any other abnormal effect. Death and observed clinical signs were recorded in the comment of datasheet for each animal in detail.

Tumor volumes were measured twice per week in two dimensions using a caliper, and the volume were expressed in mm³ using the formula: V = *0.5 a* x *b*² where *a* and *b* were the length and width of the tumor, respectively. (Tumor weight were measured at the end of study based on sponsor's request). The entire procedures of dosing as well as tumor and body weight measurement were conducted in a Laminar Flow Cabinet.

Handling of animals with BWL during the study and the human end points of the animals: The treatment can be resumed when the change in animal body weight was less than 10%. The health status of animals were analyzed based on continuous observation of the following symptoms and in terms of animal welfare. For example, marked hypoactivity and signs of muscular atrophy were observed.

Study Termination: The study was routinely terminated when the mean tumor burden in the vehicle treated control group reaches a value of 2000 mm³ or one week post the final dose, whichever comes first. Sample collection includes tumor collection, tumor weights, preservation method for fixed in formalin and processed to FFPE samples.

Summary statistics: the mean and standard error of the mean (SEM) were provided for the tumor volumes of each group at every time point. Statistical analysis of difference in tumor volume between the two comparing groups was conducted on the data obtained at the best therapeutic time point (usually after the final dose) using Independent-Samples T Test. All data were analyzed in SPSS (Statistical Product and Service Solutions) version 18.0 (IBM, Armonk, NY, U.S.). P-values were rounded to three decimal places, with the exception when raw P-values were less than 0.001, then they were stated as P<0.001. All tests were two-sided. P<0.05 was considered to be statistically significant.

Compliance: The protocol and any amendment(s) or procedures involving the care and use of animals in this study were reviewed and approved by the Institutional Animal Care and Use Committee (IACUC) of CrownBio prior to conduct. During the study, the care and use of animals were conducted in accordance with the regulations of the Association for Assessment and Accreditation of Laboratory Animal Care (AAALAC).

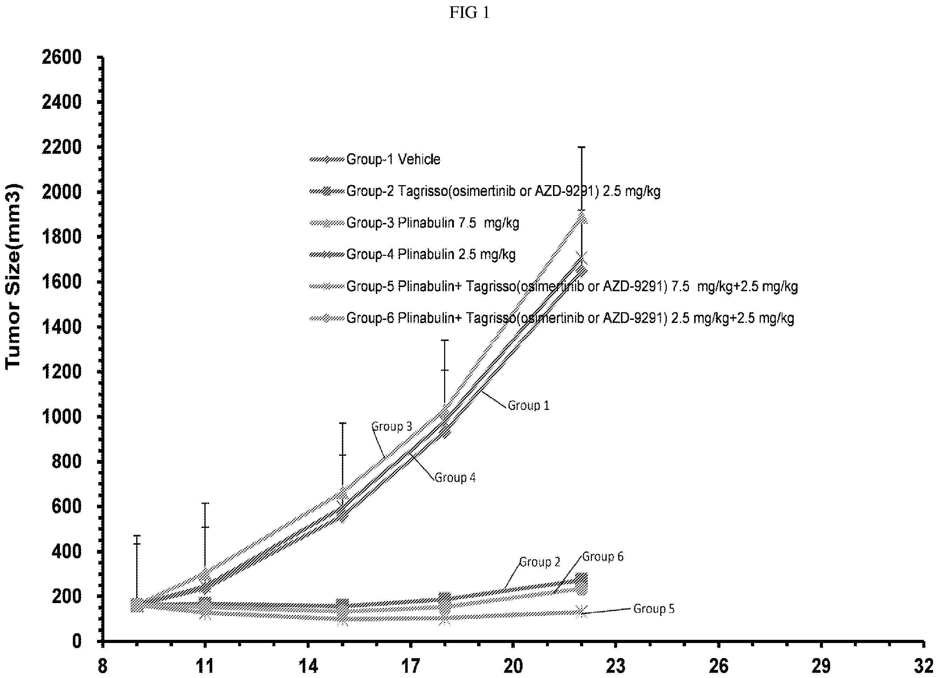

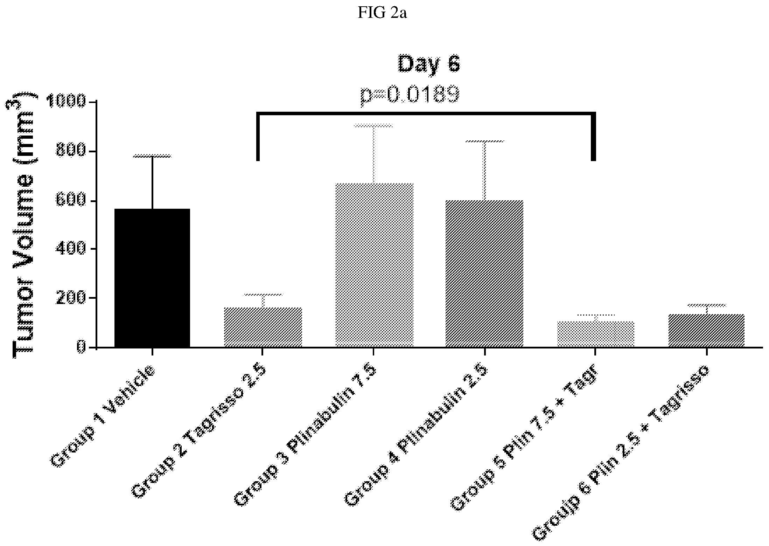

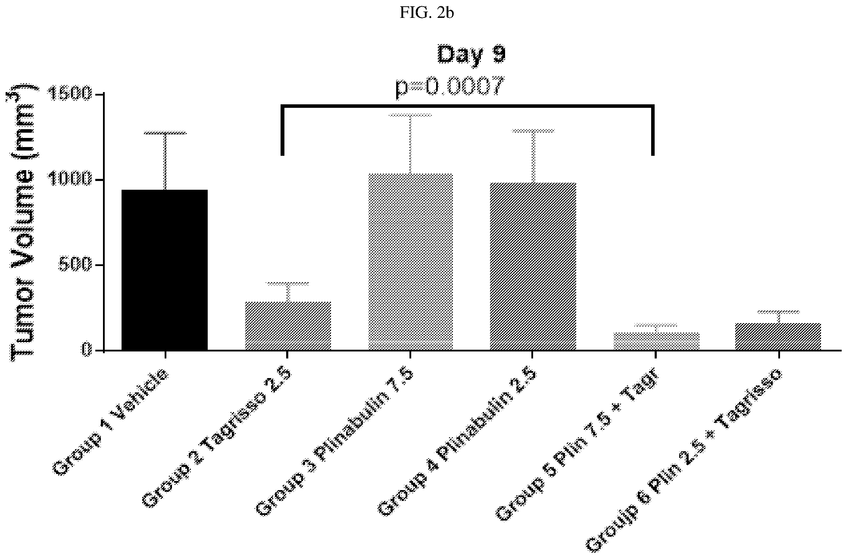

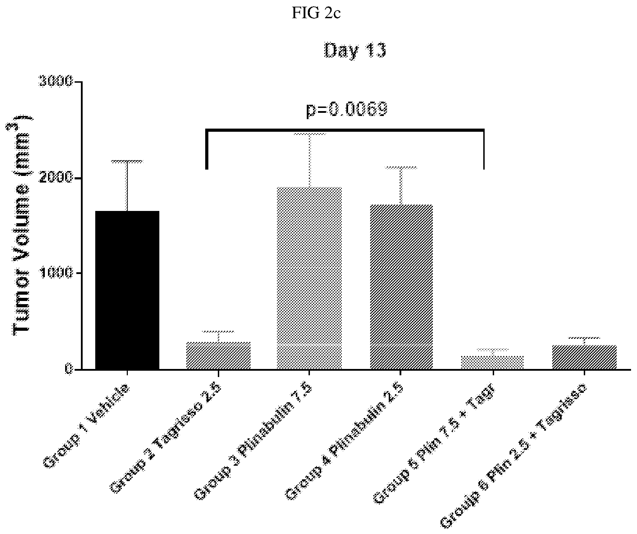

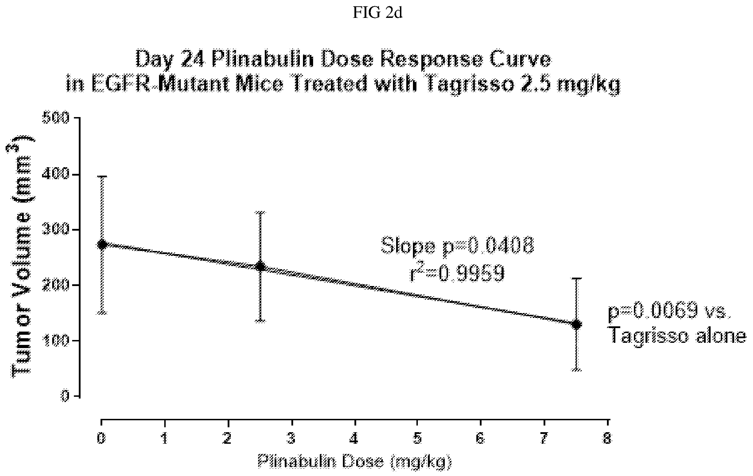

The study results are shown in figure 1 and figures 2a-2d. The tumor size changes after tumor inoculation were compared in the six test groups. Group 1 (vehicle only), Group 3 (plinabulin 7.5 mg/kg), and Group 4 (plinabulin 2.5 mg/kg) showed tumor size increases after administration of the tested formulation; Group 2 (osimertinib 2.5 mg/kg) and Group 6 (plinabulin 7.5 mg/kg + osimertinib 2.5 mg/kg) showed control over the tumor size and slight increase in tumor size after the animals were tested for 15 days; and Group 5 (plinabulin 7.5 mg/kg + osimertinib 2.5 mg/kg) showed better control of tumor size among the tested groups with no substantial tumor size increase even after 22 days.

## Claims

1. Plinabulin, or a pharmaceutically acceptable salt thereof, for use in combination with osimertinib for treating a cancer or tumor **characterized by** expression of a mutant form of an epidermal growth factor receptor (EGFR) protein, wherein the mutant form of the EGFR protein comprises an amino acid substitution T790M.

2. Plinabulin for use according to Claim 1, wherein the cancer or tumor is selected from the group consisting of squamous cell cancer, non-small cell lung cancer, glioblastoma, epithelial tumors of the head and neck, squamous carcinoma of the lung, colon cancer, endometrial carcinoma, multiple myeloma, and hepatocellular carcinoma.

3. Plinabulin, or a pharmaceutically acceptable salt thereof, for use in combination with osimertinib for inhibiting proliferation of a cell expressing a mutant form of an EGFR protein, wherein the mutant form of the EGFR protein comprises an amino acid substitution T790M.

4. Plinabulin, or a pharmaceutically acceptable salt thereof, for use in combination with osimertinib for inhibiting progression of a cancer or tumor **characterized by** expression of a mutant form of an EGFR protein in a subject, wherein the mutant form of the EGFR protein comprises an amino acid substitution T790M.

5. Plinabulin for use according to any one of Claims 1 to 4, wherein the use comprises determining whether the subject has an EGFR mutation.

6. Plinabulin for use according to any one of Claims 1 to 5, wherein the use comprises identifying whether the subject has a metastatic EGFR T790M mutation-positive tumor.

7. Plinabulin for use according to any one of Claims 1 to 6, wherein the osimertinib is administered prior to, concurrently with, or after the administration of plinabulin.

8. Plinabulin for use according to Claim 7, wherein the cancer or tumor is selected from the group consisting of squamous cell cancer, non-small cell lung cancer, glioblastoma, epithelial tumors of the head and neck, squamous carcinoma of the lung, colon cancer, endometrial carcinoma, multiple myeloma, and hepatocellular carcinoma.

9. Plinabulin for use according to Claim 8, wherein the cancer or tumor is non-small cell lung cancer.

10. A pharmaceutical composition comprising plinabulin and osimertinib.

## Patentansprüche

1. Plinabulin oder ein pharmazeutisch unbedenkliches Salz davon zur Verwendung in Kombination mit Osimertinib zur Behandlung eines Krebses oder Tumors, der durch die Expression einer mutierten Form eines EGFR(Epidermal Growth Factor Receptor)-Proteins gekennzeichnet ist, wobei die mutierte Form des EGFR-Proteins eine Aminosäuresubstitution T790M umfasst.

2. Plinabulin zur Verwendung nach Anspruch 1, wobei der Krebs bzw. Tumor aus der Gruppe bestehend aus Plattenepithelkrebs, nichtkleinzelligem Lungenkrebs, Glioblastom, Epitheltumoren des Kopfes und Halses, Plattenepithelkarzinom der Lunge, Darmkrebs, Endometriumkarzinom, multiplem Myelom und hepatozellulärem Karzinom ausgewählt ist.

3. Plinabulin oder ein pharmazeutisch unbedenkliches Salz davon zur Verwendung in Kombination mit Osimertinib zur Hemmung der Proliferation einer Zelle, die eine mutierte Form eines EGFR-Proteins exprimiert, wobei die mutierte Form des EGFR-Proteins eine Aminosäuresubstitution T790M umfasst.

4. Plinabulin oder ein pharmazeutisch unbedenkliches Salz davon zur Verwendung in Kombination mit Osimertinib zur Hemmung des Fortschreitens eines Krebses oder Tumors, der durch die Expression einer mutierten Form eines EGFR-Proteins gekennzeichnet ist, bei einem Individuum, wobei die mutierte Form des EGFR-Proteins eine Aminosäuresubstitution T790M umfasst.

5. Plinabulin zur Verwendung nach einem der Ansprüche 1 bis 4, wobei die Verwendung Bestimmen, ob bei dem Individuum eine EGFR-Mutation vorliegt, umfasst.

6. Plinabulin zur Verwendung nach einem der Ansprüche 1 bis 5, wobei die Verwendung Identifizieren, ob bei dem Individuum ein für die EGFR-T790M-Mutation positiver metastatischer Tumor vorliegt, umfasst.

7. Plinabulin zur Verwendung nach einem der Ansprüche 1 bis 6, wobei das Osimertinib vor, gleichzeitig mit oder nach der Verabreichung von Plinabulin verabreicht wird.

8. Plinabulin zur Verwendung nach Anspruch 7, wobei der Krebs bzw. Tumor aus der Gruppe bestehend aus Plattenepithelkrebs, nichtkleinzelligem Lungenkrebs, Glioblastom, Epitheltumoren des Kopfes und Halses, Plattenepithelkarzinom der Lunge, Darmkrebs, Endometriumkarzinom, multiplem Myelom und hepatozellulärem Karzinom ausgewählt ist.

9. Plinabulin zur Verwendung nach Anspruch 8, wobei es sich bei dem Krebs bzw. Tumor um nichtkleinzelligen Lungenkrebs handelt.

10. Pharmazeutische Zusammensetzung, umfassend Plinabulin und Osimertinib.

## Revendications

1. Plinabuline, ou un sel pharmaceutiquement acceptable de celle-ci, pour une utilisation en association avec l'osimertinib pour le traitement d'un cancer ou d'une tumeur **caractérisé par** l'expression d'une forme mutante d'une protéine de récepteur de facteur de croissance épidermique (EGFR), la forme mutante de la protéine EGFR comprenant une substitution d'acide aminé T790M.

2. Plinabuline selon la revendication 1, le cancer ou la tumeur étant choisi dans le groupe constitué par le carcinome épidermoïde, le cancer du poumon non à petites cellules, le glioblastome, les tumeurs épithéliales de la tête et du cou, le carcinome épidermoïde du poumon, le cancer du côlon, le carcinome de l'endomètre, le myélome multiple et le carcinome hépatocellulaire.

3. Plinabuline, ou un sel pharmaceutiquement acceptable de celle-ci, pour une utilisation en association avec l'osimertinib pour inhiber la prolifération d'une cellule exprimant une forme mutante d'une protéine EGFR, la forme mutante de la protéine EGFR comprenant une substitution d'acide aminé T790M.

4. Plinabuline, ou un sel pharmaceutiquement acceptable de celle-ci, pour une utilisation en association avec l'osimertinib pour inhiber la progression d'un cancer ou d'une tumeur, **caractérisé par** l'expression d'une forme mutante d'une protéine EGFR chez un sujet, la forme mutante de la protéine EGFR comprenant une substitution d'acide aminé T790M.

5. Plinabuline pour une utilisation selon l'une quelconque des revendications 1 à 4, l'utilisation comprenant la détermination de la présence ou non d'une mutation de l'EGFR chez le sujet.

6. Plinabuline pour une utilisation selon l'une quelconque des revendications 1 à 5, l'utilisation comprenant la détermination de la présence ou non d'une tumeur métastatique présentant une mutation T790M d'EGFR chez le sujet.

7. Plinabuline selon l'une quelconque des revendications 1 à 6, l'osimertinib étant administré avant, simultanément ou après l'administration de plinabuline.

8. Plinabuline selon la revendication 7, le cancer ou la tumeur étant choisi dans le groupe constitué par le carcinome épidermoïde, le cancer du poumon non à petites cellules, le glioblastome, les tumeurs épithéliales de la tête et du cou, le carcinome épidermoïde du poumon, le cancer du côlon, le carcinome de l'endomètre, le myélome multiple et le carcinome hépatocellulaire.

9. Plinabuline selon la revendication 8, le cancer ou la tumeur étant un cancer du poumon non à petites cellules.

10. Composition pharmaceutique comprenant de la plinabuline et de l'osimertinib.
